**Europäisches Patentamt**

**European Patent Office**

**Office européen des brevets**

(19)

(11) Veröffentlichungsnummer: **0 224 210**
**B1**

(12) **EUROPÄISCHE PATENTSCHRIFT**

(45) Veröffentlichungstag der Patentschrift: **29.08.90**

(21) Anmeldenummer: **86116137.0**

(22) Anmeldetag: **21.11.86**

(51) Int. Cl.⁵: **G 01 N 33/72, C 12 Q 1/26**

(54) Mittel zur Bestimmung von Peroxidase-Aktivität, Verfahren zur Herstellung und seine Verwendung.

(30) Priorität: **28.11.85 DE 3541978**

(43) Veröffentlichungstag der Anmeldung:
**03.06.87 Patentblatt 87/23**

(45) Bekanntmachung des Hinweises auf die Patenterteilung:
**29.08.90 Patentblatt 90/35**

(84) Benannte Vertragsstaaten:
**AT BE CH DE ES FR GB GR IT LI LU NL SE**

(56) Entgegenhaltungen:
EP-A-0 121 317
FR-A-2 295 425
US-A-4 077 772
US-A-4 340 394
US-A-4 504 579

(73) Patentinhaber: **BEHRINGWERKE**
**Aktiengesellschaft**
**Postfach 1140**
**D-3550 Marburg 1 (DE)**

(72) Erfinder: **Pauly, Hans Erwin, Dr.**
**Finkenstrasse 1**
**D-3563 Dautphetal (DE)**
Erfinder: **Schwarz, Herbert**
**Am Kirtenfeld 3**
**D-3557 Ebsdorfergrund (DE)**

(74) Vertreter: **Klein, Otto, Dr. et al**
**Hoechst AG Zentrale Patentabteilung Postfach**
**80 03 20**
**D-6230 Frankfurt am Main 80 (DE)**

**Beschreibung**

Die vorliegende Erfindung betrifft ein Mittel zur Bestimmung von Peroxidase-Aktivität durch Farbreaktion sowie ein Verfahren zu seiner Herstellung und seine Verwendung.

Eine wesentliche Voraussetzung für die Einführung von Enzymimmunoassays, die hinsichtlich ihrer Nachweisempfindlichkeit radioimmunologischen Methoden ebenbürtig waren, stellte die Verfügbarkeit von stabilen Markerenzymen und entsprechenden hochempfindlichen Farbbildungsreagenzien dar, mit denen die katalytische Aktivität dieser Markerenzyme in meßtechnisch einfacher Weise zu registieren war. Als besonders geeignete Markerenzyme erwiesen sich hierbei die Oxidoreductasen Glucoseoxidase und Peroxidase. Peroxidase-Reaktionen gehören generell zu den am häufigsten verwendeten enzymatischen Nachweisreaktionen. Alle z.B. in "Methods of Enzymatic Analysis", H. U. Bergmeyer, Ed., 3. Edition, Vol. 1, pp. 210—221, Verlag Chemie, Weinheim (1983) beschriebenen Verfahren beruhen, wie die Glucose-Bestimmung mittels Glucoseoxidase, auf der stöchiometrischen Erzeugung von Wasserstoffperoxid. Dieses kann dann in einer von Peroxidase katalysierten Oxidation eines farblosen Substrats zu einem farbigen Produkt umgesetzt werden, das einfach spektrophotometrisch quantifiziert werden kann.

Für diese von Peroxidase katalysierte Reaktion geeignete chromogene Systeme sind daher in großer Zahl untersucht und beschrieben (siehe Bergmeyer). Für die Bestimmung von Peroxidase-Aktivität in Enzymimmunoassays erfüllen nur wenige die Anforderungen, insbesondere hinsichtlich der Nachweisempfindlichkeit. Generell sollte ein chromogenes Substrat eine hohe Umsatzgeschwindigkeit erlauben und zu einem farbstabilen Produkt mit einem hohen molaren Extinktionskoeffizienten führen. Weiterhin sollten Substanzen bevorzugt werden, deren Handhabung kein gesundheitliches Risiko birgt. In kommerziellen Testkits für auf Peroxidase basierenden Enzymimmunoassays kommen vor allem o-Phenylendiamin (OPD) und 2,2'-Azinodi-(3-ethylenbenzothiazolinsulfonat-6) (ABTS) zum Einsatz. Sowohl OPD als auch ABTS sind wie die meisten Peroxidase-Substrat mutagen. Eine Gruppe der häufig verwendeten Substrate ist die des Benzidin-Typs, zu der auch Tetramethylbenzidin (TMB) gehört. TMB ist ein sicherer nichtmutagener Ersatz für carcinogene Peroxidase-Substrate des Benzidintyps wie Benzidin, Diaminobenzidin u.a. In zahlreichen Untersuchungen ergab sich kein Hinweis auf mutagene Eigenschaften dieses Benzidinderivats (Tetrahedron 30, 3299 (1976); Cancer Lett. 1, 39 (1975); J. Forensic Sci. 21, 816 (1976). TMB wurde seite 1974 von verschiedenen Anwendern zur Bestimmung der Pseudoperoxidase-Aktivität von Hämoglobin oder Cytochrome P 450 und bereits von Liem et al., Anal. Biochem. 98, 388—393 (1979) zur Detektion von Peroxidase-Aktivität in Immunkomplexen bei der Immunperoxidase-Färbetechnik verwendet. Diese Autoren weisen in ihrer Schlußfolgerung auf Seite 392 auf die guten Färbeeigenschaften, jedoch aber auch auf die geringe Löslichkeit des TMB in überlicherweise verwendeten Puffersystemen und auf die oxidative Zersetzung des TMB hin.

Bei der Verwendung von TMB in Enzymimmunoassays erweist sich jedoch die geringe Wasserlöslichkeit dieses Chromogens unter Testbedingungen als sehr einschränkend. In der niederländischen Anmeldungsschrift 8001972 der Firma Akzo und im US-Patent 4,503,143 wurden daher die Löslichkeit steigernde Maßnahmen beschrieben. In beiden Schriften wird die verbesserte Löslichkeit durch den Einsatz von organischen Lösungsmitteln erreicht. Durch Zusatz von Dimethylsulfoxid (1% v/v im gebrauchsfertigen Ansatz) erhält man gemäß den Angaben in NL 8001972 eine TMB-Konzentration von 100 mg/l, d.h. 0.42 mmol/l bzw. von 43 mg/l, d.h. 0.18 mmol/l im US-Patent 4,503,143. Demgegenüber wird üblicherweise OPD oder ABTS in 50 bis 100-fach höherer molarer Konzentration dem Enzym angeboten. Eine Variation der Konzentration von Tetraalkylbenzidin im Enzymtest zeigt, daß die für die Erreichung der maximalen Umsatzgeschwindigkeit erforderliche Sättigung der Peroxidase mit Substrat bei den oben angegebenen TMB-Konzentrationen nicht erreicht wird.

Es bestand daher der Bedarf, eine zur Bestimmung von Peroxidase geeignete, Tetraalkylbenzidin enthaltende Zubereitung zu finden, die die obenerwähnten Nachteile nicht hat. Eine solche Zubereitung, ihre Herstellung und ihre Verwendung ist Gegenstand der Erfindung.

Überraschenderweise wurde gefunden, daß eine hinsichtlich der Nachweisempfindlichkeit wesentlich verbesserte Zubereitung von Tetraalkylbenzidin erhalten werden kann, indem eine Lösung, die diese Verbindung oder eines ihrer Derivate enthält, mit einem wässrigen Puffer, der eine geeignete Wasserstoffperoxid-Konzentration enthält, auf einen pH-Wert zwischen pH 2.5 und 3.9 eingestellt wird. Obwohl für übliche Peroxidase-Substrate wie auch für TMB ein pH-Optimum der Enzymaktivität bei pH 5 bis pH 6 beschreiben ist (US—P—4,503,143; "Methods of Enzymatic Analysis", H. U. Bergmeyer, Ed., 3 Edition, Vol. III, pp. 286—293, Verlag Chemie, Weinheim (1983)), zeigt die erfindungsgemäße Zubereitung bei einem ungewöhnlich niedrigen pH-Wert, insbesondere in einem Bereich von pH 3.1 bis 3.6 eine gegenüber bisher beschriebenen Zubereitungen verbesserte Nachweisempfindlichkeit, wie in den Beispielen 2 und 3 gezeigt wird.

Gegenstand der Erfindung ist ein Mittel in Form einer flüssigen Zubereitung zum Nachweis und zur Bestimmung von Peroxidase enthaltend in einer wässrigen Lösung ein Tetraalkylbenzidin oder eines seiner Salze mit einem Gehalt von 0.6 bis 4.0 mmol/l, Peroxide als Substrat für Peroxidase mit einem Gehalt von 0,5 bis 50 mmol/l und Puffersubstanzen, die einen pH-Wert im Bereich von 2.5 bis 3.9 einstellen.

Die Stabilisierung des erfindungsgemäßen Mittels durch Penicillin oder eines seiner durch saure Hydrolyse entstehenden Abbauprodukte wird in der parallelen EP—A—O 224 813 beschrieben.

Das erfindungsgemäße Mittel kann durch Lösen einer festen Zubereitung hergestellt werden, beispielsweise eines Lyophilisats, eines Granulats oder einer Tablette, wobei die für die flüssige Zubereitung verwendeten Komponenten Tetraalkylbenzidin, Penicillin oder dessen Abbauprodukte, die Peroxide und die Puffersubstanzen in den Mengenverhältnissen enthalten sind, daß beim Auflösen in einem bestimmten Volumen wässrigen Lösungsmittels die Komponenten in den genannten Konzentrationen vorliegen. Die feste Zubereitung kann zusätzlich noch Zuschläge wie Gleitmittel, Füllstoffe und Sprengmittel, beispielsweise Polyäthylenglycol, Harnstoff und Bicarbonate enthalten.

Als Tetraalkylbenzidine sind vor allem solche verwendbar, die ein bis drei Kohlenstoffatome im Alkylteil enthalten, vorzugsweise 3,3',5,5'-Tetramethylbenzidin (TMB) oder sein Dihydrochlorid. Als Peroxide sind Natriumperborat, Wasserstoffperoxid in flüssiger Form oder als festes Harnstoffaddukt oder auch ein Wasserstoffperoxid generierendes System bestehend aus D-Glucose und Glucoseoxidase geeignet, wobei eine Konzentration von 0.5 bis 10 mmol/l eingestellt wird. Als Puffersubstanzen werden lyotrope Substanzen wie Citrate und Acetate bevorzugt.

Bei der Herstellung einer Zubereitung in flüssiger Form wird Tetraalkylbenzidin in einer ersten sauren Lösung von verdünnter Salzsäure oder von Ameisensäure mit einem pH-Wert von 1.5 bis 2.0 gelöst. Gegebenenfalls werden das Penicillin oder seine durch saure Hydrolyse entstehenden Abbauprodukte zu dieser Lösung zugesetzt.

Eine zweite, weniger saure Lösung wird hergestellt durch Lösen der Peroxide oder durch deren Eintragen im Falle der Verwendung einer Lösung von Wasserstoffperoxid, in Lösungen von beispielsweise Essigsäure oder Mononatriumbeziehungsweise Monokalium-Citrat, deren pH-Wert mit Natriumhydroxid zwischen 3 und 6 reguliert werden kann.

Durch Vermischen der beiden Lösungen in einem bestimmten Verhältnis wird die gebrauchsfertige Zubereitung erhalten.

Die Erfindung wird durch die folgenden Beispiele näher erläutert.

### Beispiele

1. Herstellung einer gebrauchsfertigen TMB-Substratzubereitung

Stammlösung 1: TMB-Dihydrochlorid wurde unter Rühren in einer Konzentration von 5 g/l, d.h. von 16 mmol/l in bidestilliertem Wasser gelöst und mit 5 normaler Salzsäure auf pH 1.5 eingestellt. Zu dieser Lösung wurde penicillin G unter Rühren in einer Endkonzentration von 200 mg/l, d.h. von 0.56 mmol/l zugesetzt.

Stammlösung 2: Zu 900 ml bidestilliertem Wasser wurden 1.4 ml Eisessig, 1.5 ml 1 normale NaOH und 250 mg, d.h. 3 mmol $H_2O_2$ Harnstoff-Wasserstoffperoxid-Addukt zugesetzt. Nach vollständigem Lösen wurde mit bidestilliertem Wasser auf 1 l aufgefüllt.

Gebrauchslösung: Ein Volumenteil der Stammlösung 1 und 10 Volumenteile der Stammlösung 2 wurden miteinander vermischt.

Die derart hergestellte Gebrauchslösung hatte einen pH-Wert von 3.3. Sie zeigte bei 650 nm eine optische Dichte von 0.025. Nach Zusatz des 5-fachen Volumens an 0.5 normaler Schwefelsäure betrug die bei 450 nm gemessene Extinktion 0.008.

2. Kinetischer Peroxidase-Aktivitätstest

Ein gemäß der Methode von Nakane und Kawaoi, J. Histochem. Cytochem. 22, 1084—1091 (1974) hergestelltes Konjugat aus Meerrettich-Peroxidase (Boehringer Mannheim, FRG) und Kaninchen Anti Human IgE-Antikörpern (Behringwerke, Marburg, FRG) wurde in phosphatgepufferter Kochsalzlösung (PBS für "phosphate buffered saline"), dem 10 mg/ml Rinderserumalbumin zugesetzt war, verdünnt. Davon wurden 20 µl in einer Küvette einem Volumen von 1 ml der gemäß Beispiel 1 hergestellten Gebrauchslösung zugesetzt. Nach dem Mischen wurde die Extinktionsänderung innerhalb der ersten Minute bei 650 nm registriert. In gleicher Weise wurde die vom Enzymkonjugat bewirkte Extinktionsänderung pro Minute einer gemäß NL 8001972, Beispiel 1 c, hergestellten Gebrauchslösung von 99 mg/l, d.h. 0.41 mmol/l TMB in einer wässrigen Lösung von 0.99% (v/v) Dimethylsulfoxid und 8.73 g/l Trinatriumcitrat × $1H_2O$, die mit Phosphorsäure auf pH 6.0 eingestellt worden war, registriert. Als Mittelwert einer Doppelbestimmung wurde für die TMB-Gebrauchslösung gemäß Beispiel 1 eine Extinktionsänderung pro Minute von 1.318 und für die TMB-Gebrauchslösung gemäß NL 800 1972 ein Wert von 0.193 erhalten.

3. Einsatz im Enzymimmunoassay zur Bestimmung von Gesamt-IgE

Mikrotestplatten aus Polystyrol (Nunc, Roskilde, Dänemark, Kat.-Nr. 262170) wurden gemäß dem von Voller et al., Bull. World Health-Organ. 53, 55—65 (1976) beschriebenen Verfahren beschichtet. Dazu wurden pro Vertiefung 150 µl einer Lösung des in Beispiel 2 angegebenen Anti IgE-Antikörpers in einer Konzentration von 10 µg/ml im von Voller et al. angegebenen Beschichtungspuffer 15—20 Stunden bei Raumtemperatur inkubiert. Nach Absaugen der Lösung und Waschen mit 1 g/l Polyoxiethylen(20)sorbitanmonolaureat, auch Tween®—20 genannt, in PBS (PBS-Tween®) wurden in 8 Vertiefungen jeweils 100 µl von verschiedenen Verdünungen eines IgE-haltigen Humanserums in PBS, dem 10 mg/ml Rinderserumalbumin zugesetzt worden war, eingebracht und 1 Stunde bei Raumtemperatur inkubiert. Die in diesen Proben durch den Verdünnungsschritt erzielten Konzentrationen an IgE betrugen 1000, 500, 100, 25 und 10 IU/ml. Nach zweimaligem Waschen mit PBS-Tween® wurden 100 µl einer geeigneten Verdünnung des in Beispiel 3 beschriebenen Konjugats in PBS-Tween®, dem 10 mg/ml Rinderserumalbumin zugesetzt worden war, in alle benutzten Vertiefungen eingebracht und 1 Stunde bei Raumtemperatur inkubiert. Nach weiterem zweimaligem Waschen wurden in je 4 der pro Konzentrationsstufe

EP 0 224 210 B1

benutzten 8 Vertiefungen je 100 µl der in Beispiel 1 beschriebenen Gebrauchslösung pipettiert und 30 Minuten bei Raumtemperatur inkubiert. Analog wurden je 100 µl der in Beispiel 3 beschriebenen TMB-Zubereitung gemäß NL 8001972 für die verbleibenden 4 Vertiefungen eingesetzt. Nach Ablauf der Inkubationsphase wurden 100 µl einer 0.5 normalen $H_2SO_4$ zugesetzt und damit die Enzymreaktion gestoppt. Die Extinktionen der erhaltenen Farblösungen wurden mit dem Gerät Behring ELISA-Processor (Behringwerke, Marburg, FRG) gemessen und sind in der Tabelle dargestellt.

TABELLE

Extinktionswerte im Sandwich-Enzymimmunoassay

|  |  | Ansatz 1 (TMB gemäß Beispiel 1) | Ansatz 2 (TMB gemäß NL 8001972) |
|---|---|---|---|
| Verdünnungen | 10 IU/ml | 0.077 | 0.021 |
| von IgE-haltigem | 25 IU/ml | 0.126 | 0.038 |
| Humanserum | 100 IU/ml | 0.332 | 0.102 |
| (siehe | 500 IU/ml | 1.018 | 0.305 |
| Beispiel 3) | 1000 IU/ml | 1.940 | 0.612 |

1 IU = 2.3 ng IgE

## Patentansprüche

1. Mittel zum Nachweis und zur Bestimmung von Peroxidase-Aktivität enthaltend in einer wässrigen Lösung ein Tetraalkylbenzidin oder eines seiner Salze, ein Peroxid oder ein Wasserstoffperoxid generierendes System und eine Puffersubstanz, dadurch gekennzeichnet, daß es eine oder mehrere Puffersubstanzen enthält, die einen pH-Wert im Bereich von 2,5 bis 3,9 einstellen, kein organisches Lösungsmittel enthält.

2. Mittel nach Anspruch 1, dadurch gekennzeichnet, daß das Tetraalkylbenzidin ein bis drei Kohlenstoffatome im Alkylteil enthält.

3. Mittel nach Anspruch 1, dadurch gekennzeichnet, daß das Tetraalkylbenzidin 3,3',5,5'-Tetramethylbenzidin ist.

4. Verfahren zur Herstellung eines Mittels gemäß Anspruch 1 zum Nachweis und zur Bestimmung von Peroxidase-Aktivität, dadurch gekennzeichnet, daß ein Tetraalkylbenzidin einer Lösung, die kein organisches Lösungsmittel enthält, von pH 1,5 bis 2 zugesetzt wird und die erhaltene Lösung vermischt wird mit einer weiteren Lösung, die ebenfalls kein organisches Lösungsmittel enthält, enthaltend Peroxid und Puffersubstanzen, die nach dem Mischen einen pH-Wert im Bereich von 2,5 bis 3,9 einstellen.

5. Verwendung des Mittels gemäß Anspruch 1 in analytischen Verfahren, in denen Peroxidase- oder Pseudoperoxidase-Aktivität nachgewiesen oder bestimmt wird.

## Revendications

1. Agent pour la mise en évidence et pour le dosage de l'activité de la peroxydase, contenant dans une solution aqueuse une tétra-alkylbenzidine ou un de ses sels, un peroxyde ou un système générateur de peroxyde d'hydrogène, et une substance tampon, caractérisé en ce qu'il contient une ou plusieurs substances tampon qui ajustent un pH dans la plage de 2,5 à 3,9, ne contient pas de solvant organique.

2. Agent selon la revendication 1, caractérisé en ce que la tétra-alkylbenzidine contient de 1 à 3 atomes de carbone dans le fragment alkyle.

3. Agent selon la revendication 1, caractérisé en ce que la tétra-alkylbenzidine est la 3,3',5,5'-tétraméthylbenzidine.

4. Procédé pour la préparation d'un agent selon la revendication 1 pour la mise en évidence et pour le dosage de l'activité de la peroxydase, caractérisé en ce que l'on ajoute une tétra-alkylbenzidine à une solution qui ne contient pas de solvant organique, à pH 1,5—2, et on mélange la solution obtenue avec une autre solution, ne contenant pas non plus de solvant organique, qui contient un peroxyde et des substances tampon qui, après le mélangeage, ajustent un pH dans la plage de 2,5 à 3,9.

5. Utilisation de l'agent selon la revendication 1 dans des procédés analytiques dans lesquels on met en évidence ou dose l'activité de la peroxydase ou de la pseudoperoxydase.

## Claims

1. An agent for the detection and for the determination of peroxidase activity, containing in an aqueous

4

solution a tetraalkylbenzidine or one of its salts, a peroxide or a system generating hydrogen peroxide, and a buffer substance, which

contains one or more buffer substances which set up a pH in the range 2.5 to 3.9, and

contains no organic solvent.

2. An agent as claimed in claim 1, in which the tetraalkylbenzidine contains one to three carbon atoms in the alkyl moiety.

3. An agent as claimed in claim 1, in which the tetraalkylbenzidine is 3,3',5,5'-tetramethylbenzidine.

4. A process for the preparation of an agent as claimed in claim 1 for the detection and for the determination of peroxidase activity, which comprises addition of a tetraalkylbenzidine to a solution which contains no organic solvent and is of pH 1.5 to 2, and mixing the resulting solution with another solution which likewise contains no organic solvent but contains peroxide and buffer substances which, after mixing, set up a pH in the range 2.5 to 3.9.

5. The use of the agent as claimed in claim 1 in analytical methods in which peroxidase or pseudoperoxidase activity is detected or determined.